# EUROPEAN PATENT SPECIFICATION

(11) **EP 2 014 655 B1**
(45) Date of publication and mention of the grant of the patent: **26.08.2009**
(21) Application number: 07108744.9
(22) Date of filing: 23.05.2007
(51) Int. Cl.: C07D 313/00, A61K 31/335, A61P 31/00

(54) **Etnangien derivatives and their use as antibiotics**
Etnangien-Derivate und Verwendung als Antibiotika
Dérivés de l'etnangien et leur application comme antibiotiques

(43) Date of publication of application: 14.01.2009
(73) Proprietor: Helmholtz-Zentrum für Infektionsforschung, 38124 Braunschweig (DE)
(72) Inventor: Menche, Dirk Dr., 38302, Wolfenbüttel (DE); Hassfeld, Jorma Dr., 10439, Berlin (DE); Arikan, Fatih, 38124, Braunschweig (DE); Jansen, Rolf Dr., 38124, Braunschweig (DE); Irschik, Herbert Dr., 38302, Wolfenbüttel (DE)
(74) Representative: Taruttis, Stefan Georg

(56) References cited:
- EP-A- 1 568 781
- DE-A1- 19 630 980

## Description

The present invention relates to novel compounds having antibiotic activity, which compounds can be used as a component of pharmaceutical compositions for use as an antibiotic and/or as an antiviral agent. Accordingly, the present invention provides the use of the novel compound for the production of a pharmaceutical composition for use as an antibiotic and/or as an antiviral agent.

Further, the present invention provides a production process including an isolation method for separating the novel compound from an aqueous mixture, e.g. from a fermentation broth.

### State of the art

DE 19630980 A1describes an antibiotic compound termed Etnangien, which is obtained by fermentation of *Sorangium cellulosum* (DSM 11028), adsorbed to Amberlite XAD adsorber resin and eluted from the adsorber resin using methanol. The methanol eluate can further be purified by extraction with acetic acid ester, dissolving in diethylether, extracting with aqueous sodium hydrogencarbonate, acidifying, and extracting with dichloromethane. After removal of dichloromethane, the residue can be purified by reverse phase chromatography using methanol/water as the eluent, extracting the aqueous phase with acetic acid ester, and drying.

The structure given for Etnangien is:

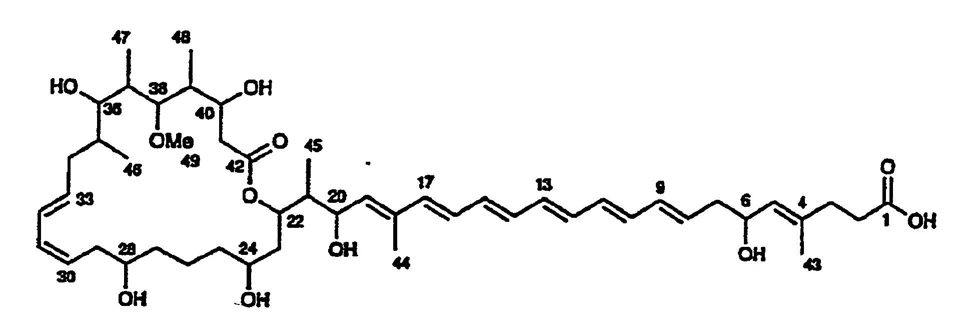

An antibacterial activity has been determined for Etnangien and indicated to be based on the inhibition of bacterial DNA- dependent RNA polymerase. However, during the development of the present invention, a compound having the known structure could not be found.

### Objects of the invention

It is an object of the present invention to provide an alternative compound to known antibiotics, especially an alternative to Etnangien, having an increased stability, especially in solution at about neutral pH-values.

A further object of the invention is to provide a production process, especially a simplified isolation and synthesis procedure, for isolating the novel compound from an aqueous composition, e.g. starting from a fermentation broth.

### General description of the invention

The present invention attains the above-mentioned objects by providing compounds **2** according to formula I, respectively. 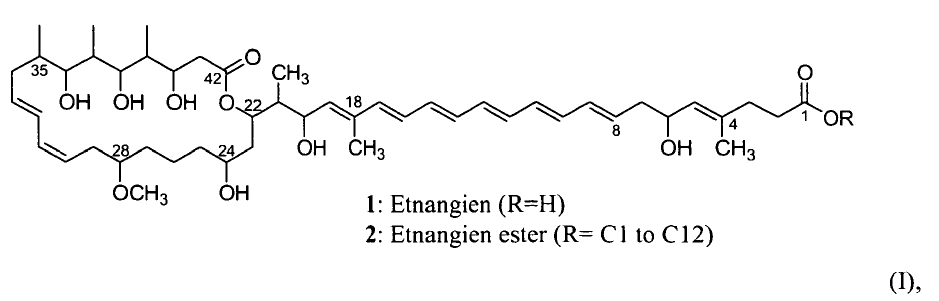 wherein R is an ester forming group linked to C-1, respectively. Although the core structure of the compounds according to the present invention differs in a hydroxyl group at C-38 instead of the methoxy group at C-38 in the compound according to DE 19630980 A1, compounds according to the present invention are presently termed Etnangien and Etnangien ester, respectively.

The ester forming group can be selected from C1 to C12 alkylene oxy, aryl oxy arylalkyloxy, aryloxy and aromatic groups, e.g. benzoxy or phenolate. Preferably, the ester is an alkylester, e.g. the methylester, ethylester, n-propylester, isopropylester, n-butylester, isobutylester, n-heptylester, n-isoheptylester, n-pentyl- or isopentylester, n-hexylester, isohexylester or a C7 to C12 linear, branched or cyclic alkylester.

The compounds according to the present invention are preferably produced by a process comprising the fermentation of *Sorangium cellulosum,* e.g. strain DSMZ 11028, and isolation of Etnangien according to structure I with R = H, i.e. the free Etnangien acid **1**. Subsequently, an ester forming group can be introduced for formal replacement of the hydroxyl group of C-1, e.g. by esterification, for example using an alkylation reaction.

The Etnangien esters according to the present invention can be obtained essentially in a quantitative manner from a reaction mixture comprising an extract of cell mass and/or, preferably combined with, adsorber resin contained in the cultivation medium of a fermentation broth from *Sorangium cellulosum.*

As a specific advantage, the Etnangien esters **2** of the invention have a significantly increased stability in comparison to Etnangien **1,** e.g. in aqueous and/or alcoholic compositions at about neutral pH, e.g. in a range of pH 5 to 8. Without exposure to light, the Etnangien esters **2** can be stored as dry matter or in solution, preferably in alcoholic solution, at room temperature without degradation for several months, e.g. in methanol, ethanol or a mixture thereof.

It has been found during the preparation of this invention, that Etnangien **1** produced by fermentation is preferably isolated in the presence of a stabiliser, e.g. 4-tert.-butyl-o-dihydroxybenzene to avoid degradation.

For production of Etnangien esters **2,** as well as for other derivatives of Etnangien **1,** a production process is preferred comprising the cultivation of a producer micro-organism, e.g. *Sorangium cellulosum,* optionally in the presence of an adsorber resin, isolation of cell mass and of the optional adsorber resin, extraction of Etnangien **1** from cell mass and/or of the adsorber resin. Preferably, further extractive and/or chromatographic processing steps follow for purification of Etnangien **1** before derivatisation reactions are performed.

Preferably, an adsorber resin having aromatic side chains, e.g. a divinylbenzene copolymer, is present during the fermentation or contacted with the fermentation broth following cultivation. The adsorber resin is preferably isolated from the fermentation broth and transferred to a chromatography column, e.g. an open or expanded bed column. The resin is preferably washed with water to remove adherent cells. For extraction of Etnangien **1,** the resin is preferably extracted with an alcohol, e.g. methanol. The alcoholic extract is buffered with aqueous ammonium acetate and concentrated, e.g. by removal of the alcohol. The residue is extracted with an organic solvent, e.g. ethyl acetate (EtOAc), which solution is preferably concentrated by removal of the organic solvent, preferably by evaporation in the presence of toluene, allowing mild evaporation conditions. This first residue can be redissolved in the organic solvent and extracted with an aqueous carbonate solution, e.g. sodium hydrogen carbonate. The aqueous phase is acidified, e.g. to pH 5, and extracted with an organic solvent, e.g. EtOAc. The organic phase is preferably partitioned with aqueous buffer solution, e.g. ammonium acetate to shift the pH to a range of 6.5. to 7.3, preferably to pH 6.8 before removal of the organic solvent, e.g. by evaporation to obtain a second residue. Preferably, evaporation is performed in the presence of toluene. The second residue contains Etnangien 1 and can be purified further, e.g. by reverse phase chromatography.

Generally, it is preferred to isolate Etnangien **1** at temperatures below 10 °C, more preferred below 5 °C, most preferred at about 0 °C. Further, it is generally preferred to add 1,2-dihydroxy-4-tert.-butylbenzene to Etnangien **1** containing preparations for enhanced stability. For storage, an alcoholic solution of Etnangien **1,** e.g. in methanol, is preferred.

### Detailed description of the invention

The present invention is now described by way of examples with reference to the figures, wherein
- Figure 1 shows a graph of the effect of etnangien **(1)** on the viability of *Micrococcus luteus,* and
- Figures 2 and 3 show measurement results of inhibition assays of nucleic acid polymerase by compounds of the invention.

### Example 1: Production of Etnangien 1

For the production of the Etnangien acid **1,** which is a preferred intermediary product in the production of derivatives of Etnangien acid **1,** e.g. for the production of Etnangien esters **2,** *Sorangium cellulosum* strain DSM 11028 was cultivated at 30 °C in an agitated bio-reactor (280 L fermentation batch) under aerobic conditions in the presence of 1% v/v Amberlite XAD 16 adsorber resin (a divinylbenzene copolymer) at 30 °C in a medium containing 0.25% soluble starch, 0.25% insoluble starch (Cerestar), 0.3% peptone from soy meal, 0.05% defatted soy meal, 8 mg/L CaCl₂ x 2H₂O 0.1% Na-Fe-EDTA. Before autoclaving, the pH was adjusted to 7.6. Glucose and magnesium sulfate were autoclaved separately and added to a final content of 0.3% and 0.1%, respectively. Inoculum cultures contained HEPES-buffer to maintain pH 7.

Starting with an inoculum of 1.5% v/v of a preculture, the stirring rate was set at 80 rpm, the aeration at 0.5 L/L medium and minute. During fermentation, the pH was kept between 7.0 and 7.8. The dissolved oxygen tension decreased from 100% to 10% over the first 7 days of cultivation; on day 8, when starch and glucose were completely metabolized, the fermentation was ended by recovering XAD resin and cell mass from the fermenter broth. Wet cell mass and adsorber resin (a total of 2.8 kg) was rinsed with water to remove adherent cells from the adsorber resin, which was then transferred to an open chromatography column (10 x 30 cm).

After elution with four bed volumes methanol (2.2 L each), the eluate was buffered with 0.1 N ammonium acetate solution (50 mL) and concentrated on a rotary evaporator at 40 °C until an oil-water mixture was obtained (2 L). This mixture was extracted four times with EtOAc. The combined organic layers were evaporated at 30 *°C in vacuo* with toluene to yield a brown oily residue, which was dissolved in 300 mL of ice-cold EtOAc and extracted four times with cold 100 mL of 2.5% sodium hydrogen carbonate solution (pH 8). Centrifugation at 0°C was used for separation of layers. After evaporation of the EtOAc layer, 17.7 g enriched neutral by-products were obtained. The combined cold aqueous layers were acidified to pH 5 using ice-cold dilute acetic acid and extracted four times with cold 250 mL EtOAc each. Combined EtOAc portions were partitioned once with cold 5% ammonium acetate (pH shift to 6.8) before evaporation. After addition of toluene, evaporation was finished in high vacuum to yield 14 g of a dark oily product. For intermediate storage, 100 mg of 1,2-dihydroxy-4-tert.-butylbenzene were added as a stabiliser to a solution of the dark oily product in 200 mL methanol and kept at -70 °C. Following further purification by reverse phase chromatography, Etnangien 1 could be obtained as a yellow residue.

All previous and following processing steps were performed under protection from light, at least using brown glassware in order to avoid isomerisation of the polyene side chain.

Negative FABMS of Etnangien **1** gave a molecular ion [M-H]⁻ at *m*/*z* 839 and high-resolution FABMS revealed the molecular formula as C₄₉H₇₆O₁₁ in accordance with the ¹³C-NMR and DEPT spectra. The structure implied 12 double bond equivalents, which in combination with the yellow colour and the UV spectrum showing four bands at 323 to 376 nm suggested a hexaene structural element for Etnangien **1.** The structure was analysed using well resolved NMR spectra in acetone-d₆. Signals for each carbon atom were detected in the ¹³C-NMR with their multiplicities sorted by the DEPT spectrum and correlated with the signals of their directly bound protons from ¹H-¹³C-HMQC-NMR data. On the basis of their distinct correlation signals in the ¹H-¹H-COSY-NMR spectrum, five of the six remaining hetero atom-bound protons were assigned as OH-protons to their respective oxymethine carbons (C-6, C-20, C-24, C-36, and C-40). H/D exchange with methanol-d₄ removed the OH-signals and caused solvent shifts, especially among the methyl group signals. When tracing the correlation signals from methylene H₂-41 at δ_{H} 2.4, a continuous sequence of signals could be followed to a group of three aliphatic methylene groups around δ_{H} 1,31 to 1,46 (C-25 to C-27) within the section of the molecule spanning C-1 to C-12, which section is termed section B, with section A of the molecule spanning C-44 to C-42. As their overlapping multiplets only showed one single further correlation, section A could be assigned to a further extended region, following the direct correlations of H-24 to H-19, and finally onward via a COSY long-range correlation to the methyl group H₃-44. The low-field acylation shift of the oxymethine H-22 (δ_{H} 5.44) and the ¹H-¹³C-HMBC signals of the carboxyl signal, C-42 (δ_{H} 173.6 with H-22 (besides correlations with H-41, H-40) indicated the lactone ring closure within section A. As a result of mutual HMBC signals, the only methyl group was assigned to the C-28 oxymethine.

The structural details of molecule section B were elucidated starting from the olefin proton H-5 (δ_{H} 5.24) in two directions. According to its weak long-range correlations in the COSY spectrum, the Δ⁴ double bond was found to be substituted by the methyl H₃-43 group (δ_{H} 1.66) and the methylene H₂-3 group (δ_{H} 2.27). The latter only has one strong COSY correlation signal with the methylene H₂-2 group (δ_{H} 2.38), which carries the second carboxyl group (C-1), as indicated by the ¹H-¹³C correlation signals in the HMBC-NMR spectrum between the carboxyl signal, C-1 (δc 174.17) and H- and H-3. A correlation with the acid proton, which is the only H/D exchangeable proton remaining in the molecular formula, was not observed. These signals are often too broad for detection in 1D ¹H-NMR spectra.

Molecule section B was identified in the opposite direction through observation of strong vicinal COSY correlation signals from H-5 to the AB system of two olefin protons, H-10 and H-11 (δ_{H} 6.24 and 6.25), exhibiting no other distinct COSY-correlations besides signals overlapping with the remaining multiplet of six olefin carbons (C-12* to C-17* around δ_{H} 6.35). The position of H-17 within the multiplet was detected in the *J*-resolved ¹H-NMR spectrum from its doublet signal at δ_{H} 6.33 (*J*=18 Hz). Exactly at that chemical shift value, a proton of the polyene multiplet was correlated in the HMQC spectrum t the methine carbon signal with the highest shift (δ_{C} 138.3), which was identified as the C-17 signal independently by strong correlations in the HMBC spectrum with H-19 and methyl group H₃-44, establishing the connectivity of molecule sections A and B.

The configuration of double bonds could in part be derived from coupling constants, with *E* configuration indicating vicinal coupling constants of about 15 Hz being observed for *J*_{8,9} and *J*_{32,33}. The Z configuration of the Δ^{30,31} double bond followed from a vicinal coupling *J*_{30,31} of 11 Hz. The configuration of both methyl substituted double bonds (Δ^{4,5} and Δ^{18,19}) was evident from their correlations in the ROESY-NMR spectrum: firstly, methyl protons H₃-43 correlated with H-6 and H-3 and H-2 on one side, and H-3 correlated with H-5 on the opposite side and, secondly, methyl protons H₃-44 correlated with H-20 and at least one of the hexaene protons while H-19 correlated with a proton at δ_{H} 6.33, which was previously shown to be H-17. The all-*E* configuration of the remaining double bonds of the hexaene unit was suggested by the UV spectrum with bands at 323, 339, 356 and 376 nm, and could be deduced indirectly from the almost complete overlap of their ¹H-NMR signals while a dispersion of their ¹H-NMR signals would be expected if one double bond were in the Z configuration. Although part of the lactone ring, the 30Z and 32E double bonds of the diene adopted an s-*trans* spatial arrangement (*J*_{31,32}= 11 Hz), as indicated by the ROESY signals between H-29 and H-32 and H-31 and H-33.

### Example 2: Production of Etnangien ester 2

Preferably, 4-tert.-butyl-1,2-dihydroxybenzene was present in all compositions comprising Etnangien to act as a stabiliser before esterification. For isolation of Etnangien **1,** i.e. the non-derivatised precursor, reversed phase chromatography using methanol/ammonium acetate buffer eluents could be used on the product obtainable according to Example 1.

For production of Etnangien ester **2** according to the present invention, derivatisation by introduction of the group substituting the oxygen atom linked to C1 of the original hydroxyl group from Etnangien **1** was performed within the residual crude oil from the concentration of the extract of adsorber resin and/or *Soragium cellulosum* cell mass as described in Example 1.

In the case of alkylesters, diazoalkanes are preferred reagents for the introduction of the alkyl to generate the alkyl ester. In the case of Etnangien methylester, diazomethane is contacted with the crude oil containing Etnangien **1.** When using diazoalkanes, essentially no isomerisation of the polyene side chain of Etnangien **1** was observed.

Preferably, the alkylation agent is prepared freshly as a reagent solution, which is washed prior to use with a buffer at pH 9.
Surprisingly, the esterification of Etnangien **1** occurs to a much larger extent than the esterification of another major metabolite, e.g. icumazol, by the alkylation agent, resulting in nor or insignificant contamination of esterification product of Etnangien **1** by derivatives of icumazol.

Analysis of the alkylation reaction products by HPLC showed that the ester formation essentially proceeded quantitatively. Etnangien alkylester could be isolated by flash chromatography on silica gel using dichloromethane/methanol at 12:1 to 10:1, followed by preparative reverse phase HPLC using methanol/water at 79:21.

This combined extraction process of the original Etnangien and the esterification in a mixture with the residual crude oil from the concentration of the extract of adsorber resin and/or *Soragium cellulosum* cell mass, e.g. the alkylation using alkylating agent, has the advantage of circumventing laborious isolation protocols that were hitherto necessary to purify Etnangien. In particular, the esterification, e.g. alkylation, of Etnangien in the concentrated methanol extract of wet cell mass and adsorber resin can be performed without presence of added stabilisers and without separation of Etnangien from other components of the residual crude oil, allowing the intermediary extraction and concentration of Etnangien and its esterification, e.g. alkylation, to the Etnangien ester to proceed in the absence of added stabilizers and without laborious isolation of the intermediary Etnangien.

For the Etnangien methylester, the following ¹H - and ¹³C-NMR data were found:

**Table 1: ¹H - and ¹³C-NMR data of Etnangien methylester**

| No | ¹³C^{b} | ¹H mult., *J* (Hz) |
|---|---|---|
| 1 | 174.17 | - |
| 2 | 33.02 | 2.38 m |
| 3 | 35.18 | 2.27 m |
| 4 | 136.15 | - |
| 5 | 129.96 | 5.25 d, 8.3 |
| 6 | 68.40 | 4.37 ddd, 8.4, 6.8, 6.8 |
| 7 | 42.36 | 2.22 m / 2.33, ddd, 14.9, 7.5, 6.8 |
| 8 | 132.63 | 5.76 ddd, 14.9, 6.8, 7.5 |
| 9 | 133.47 | 6.16 dd, 14.9, 10.4 |
| 10 | 132.14 | 6.25 m |
| 11 | 134.36^{c} | 6.24 m |
| 12 | 134.13^{c} | 6.31 m |
| 13 | 134.04^{c} | 6.35 m |
| 14 | 133.97^{c} | 6.35 m |
| 15 | 133.56^{c} | 6.35 m |
| 16 | 129.48 | 6.31 m |
| 17 | 138.30 | 6.37 m |
| 18 | 135.86 | - |
| 19 | 135.54 | 5.53 d, 9.0 |
| 20 | 69.55 | 4.39 dd, 8.7, 8.3 |
| 21 | 43.77 | 1.98 m |
| 22 | 73.48 | 5.44 ddd, 8.5, 4.4, 4.4 |
| 23 | 38.68 | 1.65 m / 1.65 m |
| 24 | 67.86 | 3.51 m |
| 25 | 39.18 | 1.31 m/1.42m |
| 26 | 22.16 | 1.31 m/1.46m |
| 27 | 33.16 | 1.41 m |
| 28 | 81.13 | 3.25 m |
| 29 | 30.72 | 2.37m/2.44m |
| 30 | 126.03 | 5.35 m |
| 31 | 130.90 | 6.06 dd, 10.9, 10.9 |
| 32 | 128.29 | 6.36 m |
| 33 | 133.31 | 5.71 ddd, 14.9, 8.5, 6.2 |
| 34 | 37.65 | 1.95 m / 2.23 m |
| 35 | 36.54 | 1.75 m |
| 36 | 79.35 | 3.44 dd, 6.8, 3.8 |
| 37 | 37.30 | 1.84 m |
| 38 | 76.90 | 3.61 dd, 7.9, 2.6 |
| 39 | 42.83 | 1.87 m |
| 40 | 68.97 | 4.17 dt, 5.5, 5.5 |
| 41 | 38.61 | 2.40 (2H) d, 5.48 |
| 42 | 173.64 | - |
| Me-4 | 16.68 | 1.66 d, 0.7 |
| Me-18 | 13.22 | 1.82 d, 0.8 |
| Me-21 | 11.12 | 0.84 d, 1.0 |
| Me-35 | 15.08 | 0.92 d, 6.4 |
| Me-37 | 7.18 | 0.98 d, 6.8 |
| Me-39 | 10.94 | 0.97 d, 7.0 |
| OMe-28 | 56.39 | 3.29 |
| CO₂Me | 51.67 | 3.61 s |

| | | |
|---|---|---|
| ^{a} Spectra were recorded in acetone-D6 at 600 MHz (¹H-NMR) and 151 MHz (¹³C-NMR). ^{b} Assignments of ¹³C were achieved by HMQC and HMBC experiments. ^{c} Signals may be interchanged. | | |

For the Etnangien methylester, a colourless amorphous solid, the following properties were measured: [α] ²²_{D} +17.4° (c 0.38, MeOH); IR (film) vₘₐₓ 3018, 2967, 1715, 1094 cm⁻¹; UV (MeOH) λₘₐₓ 375, 355, 338, 323, 226, 235 nm; HRESI (+) MS 877.5443 [(M+ Na)⁺, C₅₀H₇₈O₁₁Na requires 877.5442].

### Example 3: Antibacterial activity of Etnangien ester

Antibiotic activity, which is assumed to be based on the inhibition of the RNA polymerase, was tested against several gram-positive and gram-negative bacteria and *Saccharomyces cerevisiae,* including a Rifampicin resistant *Staphylococcus aureus* clinical isolate.

The minimal inhibitory concentrations (MIC) are shown in following table 2 and demonstrate that the Etnangien ester has an antibiotic activity comparable to that of the non-derivatized Etnangien. The microorganisms tested were grown on 1% peptone, 0.1% meat extract, 0.1% yeast extract medium containing 1.5% agar at pH 7.0. MIC values were determined by serial dilution.

**Table 2: Antimicrobial activity of Etnangien methylester in comparison to Etnangien acid 1**

| Test organism | MIC (µg/mL) Etnangien methylester | MIC (µg/mL) Etnangien |
|---|---|---|
| *Staphylococcus aureus* | 2.5 | 1.0 |
| *S. aureus,* Rifampicin resistant | 3.1 | 0.62 |
| *Streptococcus faecalis* | | 2 |
| *Bacillus subtilis* | 20 | 10 |
| *Bacillus megaterium* | | 20 |
| *Corynebacterium glutamicum* (DSM 20300) | 0.24 | 0.03 |
| *Corynebacterium mediolanum* | | 0.06 |
| *Mycobacterium phlei* | | 0.12 |
| *Mycobacterium diemhoferii* (DSM 43218) | | 0.12 |
| *Mycobacterium smegmatis* (DSM 43856) | | 1 |
| *Nocardia corallina* | 0.12 | 0.12 |
| *Micrococcus luteus* | 0.39 | 0.06 |
| *E. coli* | >80 | >80 |
| *Saccharomyces cerevisiae* | >40 | >80 |
| *Rhodutorula glutinis* | | >80 |

For cultivated mouse fibroblast cells L929, an IC₅₀ of 74 µg/mL was determined.

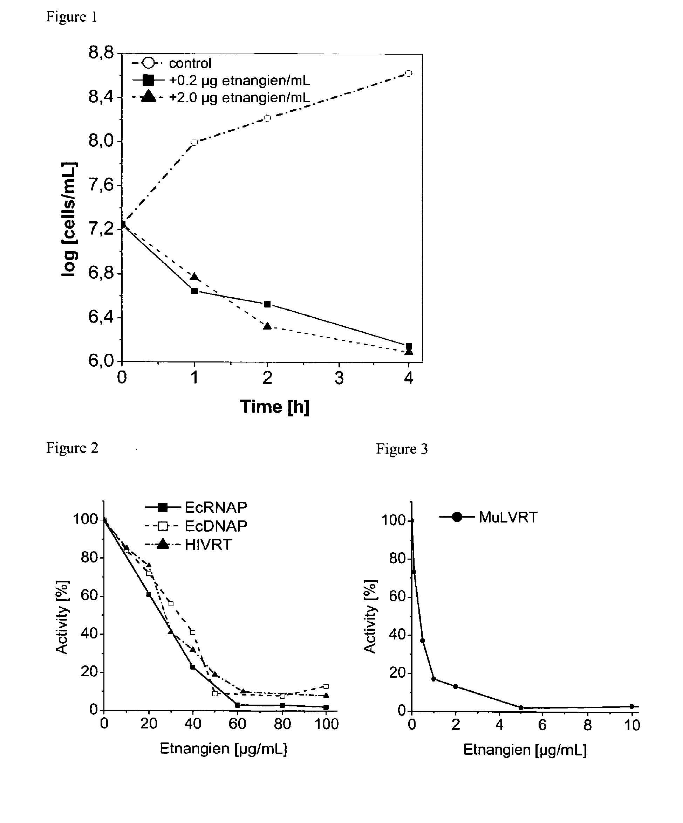

The effect of Etnangien **1** on the viability of *M. luteus* in an *in vitro* assay is shown in Figure 1. Cells were suspended in nutrient broth and incubated at 30 °C. At t = 0 the culture was divided and Etnangien **1** or methanol for comparison was added. At different times aliquots were diluted and spread on agar plates. Colonies were counted after 24 h. Similar results were found for the Etnangien esters of the invention.

Accordingly, the compounds of the invention have antibacterial activity against a large variety of species, especially against *Staphylococcus,* e.g. *S. aureus, Mycobacterium* spp. and *Nocardia.* The IC₅₀ against cultivated cells indicates that these compounds are suitable for medical use.

### Example 4: Antiviral activity of Etnangien

In addition to the use of Etnangien **1** as an antibacterial agent, it was found in *in vitro* assays that Etnangien 1 is a potent inhibitor of viral reverse transcriptase, exemplified by HIV and Moloney murine leukemia virus reverse transcriptases, the latter being inhibited completely at concentrations of 5 µg/mL Etnangien **1.** Similar antiviral activity is found for the Etnangien esters according to the invention.

Measurement results showing the nucleic acid polymerase inhibition by Etnangien **1** are shown in Figure 2 for *E. coli* RNA polymerase (EcRNAP), *E. coli* DNA polymerase (EcDNAP), and HIV I reverse transcriptase (HIVRT), and in Figure 3 for Moloney murine leukemia virus reverse transcriptase (MuLVRT). *In vitro* assays were performed according to Richardson et al. (J. Biol. Chem. 222-232 (1964)) and Houts et al. (J. Virol. 517-522(1979)). Maximum inhibition for *E. coli* polymerases was found for concentrations of 50 to 60 µg/mL, although *E. coli* itself was rather resistant. Of the retroviral reverse polymerases, the Moloney murine leukemia virus reverse transcriptase was the most sensitive tested with a maximum inhibition at 5 µg/mL Etnangien **1** and comparable values for the Etnangien esters.

Due to the low cytotoxic effect of Etnangien **1,** Etnangien and its derivatives, especially its esters, are preferred compounds for use as antiviral agents, and for use in the production of pharmaceutical compositions having antiviral activity. Preferably, the antiviral activity is directed against retrovirus, e.g. against HIV.

## Claims

1. Compound comprising a structure according to formula I 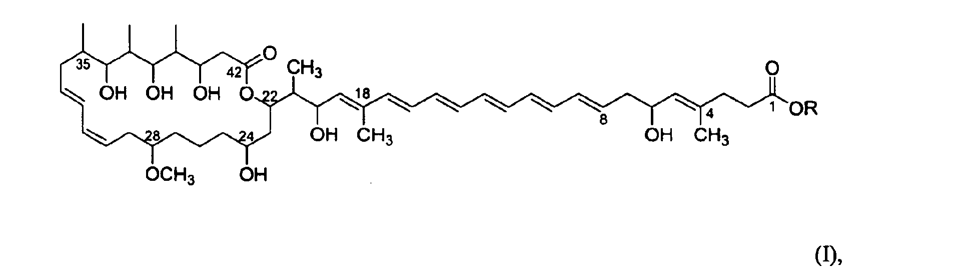 **characterized in that** R is an ester forming group.

2. Compound according to claim 1, **characterized in that** the ester forming group is selected from the group comprising C1 to C12 alkylene oxy, aryl oxy arylalkyloxy aryloxy groups, benzoxy or phenolate groups, methyl, ethyl, n-propyl, isopropyl, n-butyl, isobutyl, n-heptyl, n-isoheptyl, n-pentyl- or isopentyl, n-hexyl, isohexyl or a C7 to C12 linear, branched or cyclic alkyl.

3. Compound according to claim 3, **characterized in that** the ester forming group is a methyl group.

4. Use of a compound according to one of the preceding claims comprising a structure according to formula I 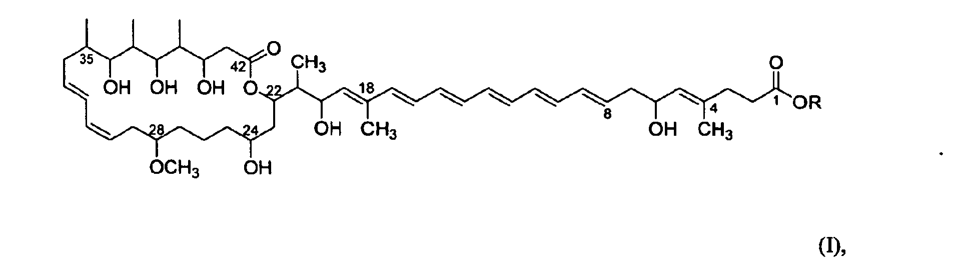 wherein R is an ester forming group for the production of a pharmaceutical composition.

5. Use of a compound according to according to claim 4, **characterized in that** the ester forming group is selected from the group comprising C1 to C12 alkylene oxy, aryl oxy arylalkyloxy aryloxy groups, benzoxy or phenolate groups, methyl, ethyl, n-propyl, isopropyl, n-butyl, isobutyl, n-heptyl, n-isoheptyl, n-pentyl- or isopentyl, n-hexyl, isohexyl or a C7 to C12 linear, branched or cyclic alkyl.

6. Use according to claim 5, **characterized in that** the pharmaceutical composition has antibacterial, antiviral and/or anti-retroviral activity.

7. Process for producing a compound comprising a structure according to formula I 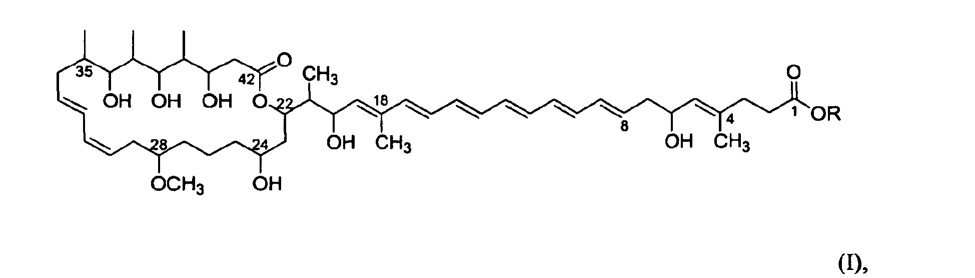 wherein R is an ester forming group according to one of claims 1 to 3 or for use according to one of claims 4 and 5, comprising the cultivation of *Sorangium cellulosum,* comprising the steps of
collecting the cell mass,
extracting the cell mass,
concentrating the extract, **characterized by** the
formation of an ester group at C1.

8. Process according to claim 7, wherein extraction of the cell mass is with methanol, concentrating the extract is by evaporation of the methanol.

9. Process according to one of claims 7 to 8, **characterized in that** the cultivation is in the presence of a resin, and the resin is subjected to collecting and extracting in admixture with the cell mass.

10. Process according to one of claims 7 to 9, **characterized in that** the cultivation is in the presence of a resin or a resin is contacted with the fermentation broth following cultivation, and the resin is separated from the fermentation broth and extracted with an alcohol and separated from the alcoholic extract, followed by removal of the alcohol from the alcoholic extract to produce a first residue, and extracting the residue with an organic solvent, partitioning of the organic solvent extract with an aqueous buffer solution until shifting the pH of the organic solvent extract to pH 6.5 to 7.3, and removal of the organic solvent to produce a second residue containing Etnangien (1).

11. Process according to claim 10, **characterized in that** the alcohol is methanol, the organic solvent is ethyl acetate, and removal of the organic solvent is in the presence of toluene.

12. Process according to one of claims 7 to 11, **characterized in that** the formation of the ester group is by contacting an alkylating agent with a residual crude oil obtained from acidification of the concentrated extract, extraction of the acidified extract with an organic solvent, separation of the organic solvent phase and removal of the solvent.

13. Process according to claim 12, **characterized in that** the organic solvent is ethyl acetate.

14. Process according to one of claims 7 to 13, **characterized in that** the alkylating agent is selected from a C1- to C12- diazoalkyl.

## Patentansprüche

1. Verbindung, die eine Struktur gemäß Formel I umfasst 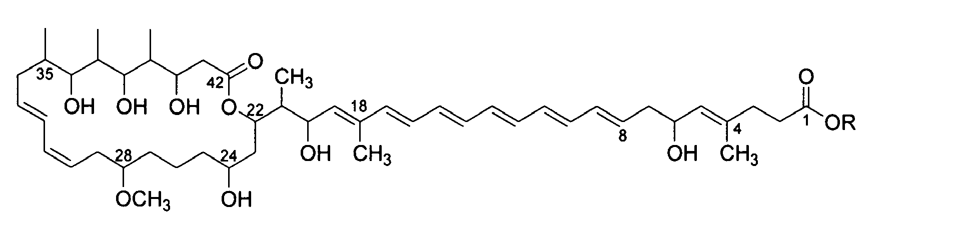 (I), **dadurch gekennzeichnet, dass** R eine esterbildende Gruppe ist.

2. Verbindung nach Anspruch 1, **dadurch gekennzeichnet, dass** die esterbildende Gruppe aus der Gruppe ausgewählt ist, die C1- bis C12-Alkylenoxy-, Aryloxy-, Arylalkyloxy, Aryloxy-Gruppen, Benzoxy- oder Phenolat- Gruppen, Methyl-, Ethyl-, n-Propyl-, Isopropyl-, n-Butyl-, Isobutyl, n-Heptyl, n-Isoheptyl, n-Pentyl- oder Isopentyl, n-Hexyl, Isohexyl oder ein C7 bis C12 lineares, verzweigtes oder zyklisches Alkyl umfasst.

3. Verbindung nach Anspruch 3, **dadurch gekennzeichnet, dass** die esterbildende Gruppe eine Methylgruppe ist.

4. Verwendung einer Verbindung nach einem der voranstehenden Ansprüche, die eine Struktur gemäß Formel I umfasst 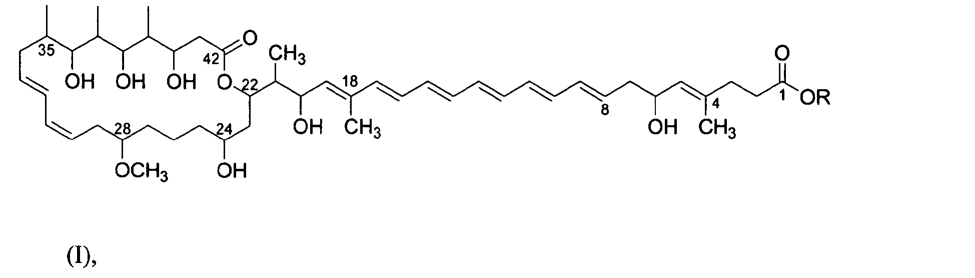 bei der R eine esterbildende Gruppe ist, zur Herstellung einer pharmazeutischen Zusammensetzung.

5. Verwendung einer Verbindung nach Anspruch 4, **dadurch gekennzeichnet, dass** die esterbildende Gruppe aus der Gruppe ausgewählt ist, die C1- bis C12-Alkylenoxy-, Aryloxy-, Arylalkyloxy-, Aryloxy-Gruppen, Benzoxy- oder Phenolat- Gruppen, Methyl-, Ethyl-, n-Propyl-, Isopropyl-, n-Butyl-, Isobutyl, n-Heptyl, n-Isoheptyl, n-Pentyl- oder Isopentyl, n-Hexyl, Isohexyl oder ein C7 bis C12 lineares, verzweigtes oder zyklisches Alkyl umfasst.

6. Verwendung nach Anspruch 5, **dadurch gekennzeichnet, dass** die pharmazeutische Zusammensetzung antibakterielle, antivirale und/oder anti-retrovirale Aktivität aufweist.

7. Verfahren zur Herstellung einer Verbindung, die eine Struktur gemäß Formel I umfasst 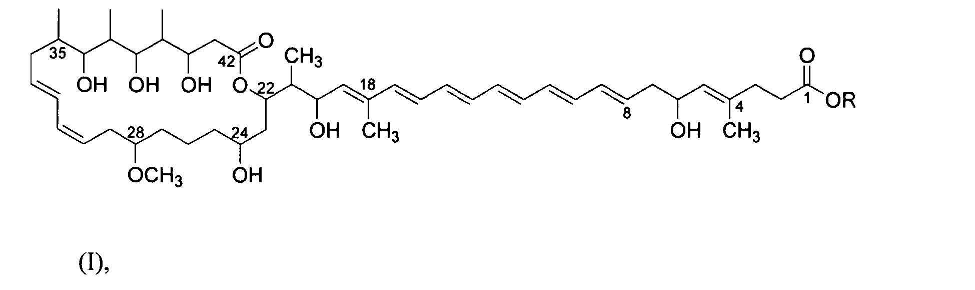 bei der R eine esterbildende Gruppe ist nach einem der Ansprüche 1 bis 3, oder zur Verwendung nach einem der Ansprüche 4 und 5, dass die Kultivierung von *Sorangium cellulosum* umfasst, umfassend die Schritte
Sammeln der Zellmasse,
Extrahieren der Zellmasse,
Konzentrieren des Extrakts, **gekennzeichnet durch** die
Bildung einer Estergruppe an C1.

8. Verfahren nach Anspruch 7, bei dem die Extraktion der Zellmasse mit Methanol ist, und Konzentrieren des Extrakts durch Verdampfen des Methanols ist.

9. Verfahren nach einem der Ansprüche 7 bis 8, **dadurch gekennzeichnet, dass** die Kultivierung in Gegenwart eines Harzes ist, und dass das Harz dem Sammeln und Extrahieren in Mischung mit der Zellmasse unterworfen ist.

10. Verfahren nach einem Ansprüche 7 bis 9, **gekennzeichnet dadurch, dass** die Kultivierung in Gegenwart eines Harzes ist oder ein Harz mit der Fermentationsbrühe im Anschluss an die Kultivierung kontaktiert wird, und das Harz von der Fermentationsbrühe extrahiert und mit einem Alkohol extrahiert wird und von dem alkoholischen Extrakt abgetrennt wird, gefolgt von der Entfernung des Alkohols aus dem alkoholischen Extrakt, um einen ersten Rückstand zu erzeugen, und Extrahieren des Rückstands mit einem organischen Lösungsmittel, Verteilen des organischen Lösungsmittelextrakts mit einer wässrigen Pufferlösung bis zum Verschieben des pHs des organischen Lösungsmittelextrakts auf pH 6,5 bis 7,3, und Entfernen des organischen Lösungsmittels, um einen zweiten Rückstand herzustellen, der Etnangien (1) enthält.

11. Verfahren nach Anspruch 10, **dadurch gekennzeichnet, dass** der Alkohol Methanol ist, das organische Lösungsmittel Ethylacetat ist, und das Entfernen des organischen Lösungsmittels in Gegenwart von Toluol erfolgt.

12. Verfahren nach einem der Ansprüche 7 bis 11, **dadurch gekennzeichnet, dass** die Bildung der Estergruppe durch Kontaktieren eines Alkylierungsmittels mit einem zurückbleibenden rohen Öl erfolgt, das vom Ansäuern des konzentrierten Extrakts erhalten wird, Extrahieren des angesäuerten Extrakts mit einem organischen Lösungsmittel, Abtrennung der organischen Lösungsmittelphase und Entfernen des Lösungsmittels.

13. Verfahren nach Anspruch 12, **dadurch gekennzeichnet, dass** das organische Lösungsmittel Ethylacetat ist.

14. Verfahren nach einem der Ansprüche 7 bis 13, **dadurch gekennzeichnet, dass** das Alkylierungmittel unter einem C1- bis C12 - Diazoalkyl ausgewählt ist.

## Revendications

1. Composé comprenant une structure selon la formule I 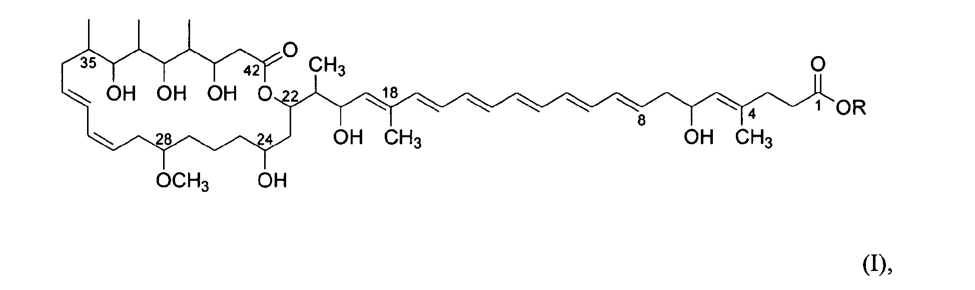 **caractérisé par le fait que** R est un groupe formant un ester.

2. Composé selon la revendication 1, **caractérisé par le fait que** le groupe formant l'ester est sélectionné à partir du groupe comprenant les alkylène-oxy C1 à C12, les groupes aryle- oxy- arylalkyloxy- aryloxy-, benzoxy- ou phénolate, méthyle, éthyle, n-propyle, isopropyle, n-butyle, isobutyle, n-heptyle, n-isoheptyle, n-pentyle- ou isopentyle, n-hexyle, isohexyle ou un alkyle C7 à C12 linéaire, branché ou cyclique.

3. Composé selon la revendication 3, **caractérisé par le fait que** le groupe formant l'ester est un groupe méthyle.

4. Utilisation d'un composé selon une des revendications précédentes comprenant une structure selon la formule I 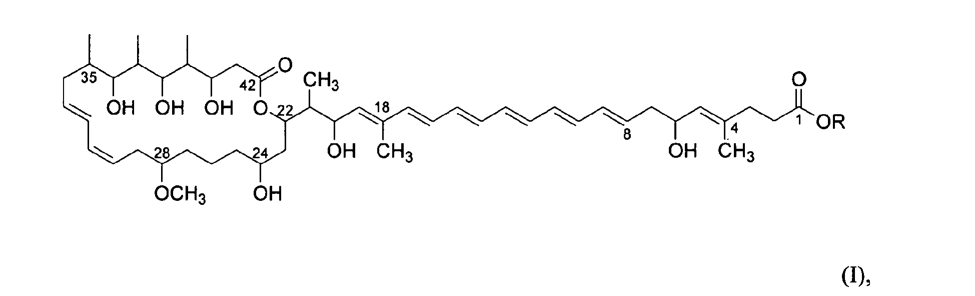 dans lequel R est un groupe formant un ester pour la production d'une composition pharmaceutique.

5. Utilisation d'un composé selon la revendication 4, **caractérisé par le fait que** le groupe formant un ester est sélectionné à partir du groupe comprenant l'alkylène-oxy C1 à C12, les groupes aryle- oxy- arylalkyloxy- aryloxy-, benzoxy- ou phénolate, méthyle, éthyle, n-propyle, isopropyle, n-butyle, isobutyle, n-heptyle, n-isoheptyle, n-pentyle- ou isopentyle, n-hexyle, isohexyle ou un alkyle C7 à C12 linéaire, branché ou cyclique.

6. Utilisation selon la revendication 5, **caractérisé par le fait que** la composition pharmaceutique a une activité antibactérienne, antivirale et/ou antirétrovirale.

7. Processus destiné à la production d'un composé comprenant une structure selon la formule I 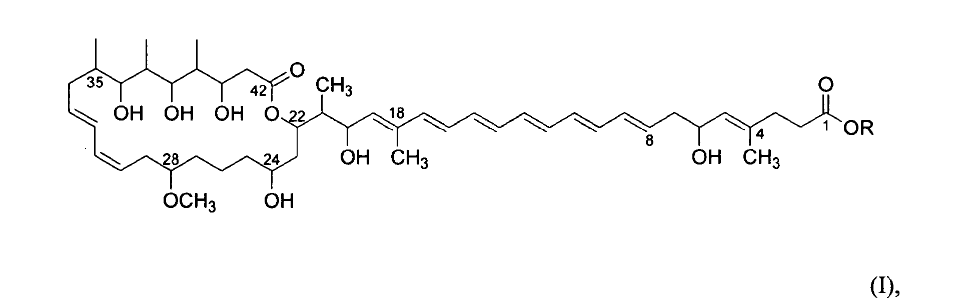 dans lequel R est un groupe formant un ester selon une des revendications 1 à 3 ou pour une utilisation selon une des revendications 4 et 5, comprenant la culture de *Sorangium cellulosum,* comprenant les étapes de
collection de la masse de la cellule,
extraction de la masse de la cellule,
concentration de l'extrait, **caractérisée par** la
formation d'un groupe d'ester à C1.

8. Processus selon la revendication 7, dans lequel l'extraction de la masse de la cellule est faite en recourant au méthanol, la concentration de l'extrait se faisant par évaporation du méthanol.

9. Processus selon une des revendications 7 à 8, **caractérisé par le fait que** la culture se fait en présence d'une résine, la résine étant sujette à la collecte et à l'extraction en mélange ayant reçu l'ajout de la masse de la cellule.

10. Processus selon une des revendications 7 à 9, **caractérisé par le fait que** la culture se fait en présence d'une résine, ou dans laquelle une résine est contactée avec le bouillon de fermentation suivant la culture, la résine étant séparée du bouillon de fermentation et extraite avec de l'alcool, et séparée de l'extrait alcoolique, opération suivie du retrait de l'alcool de l'extrait alcoolique afin de produire un premier résidu, et de l'extraction du résidu avec un solvant organique, en partageant l'extrait du solvant organique avec une solution tampon aqueuse jusqu'à déplacer le pH de l'extrait du solvant organique de pH 6,5 à 7,3, et en enlevant le solvant organique afin de produire un second résidu contenant de l'etnangien (1).

11. Processus selon la revendication 10, **caractérisé par le fait que** l'alcool est du méthanol, le solvant organique étant de l'acétate d'éthyle, et l'enlèvement du solvant organique se faisant en présence de toluène.

12. Processus selon une des revendications 7 à 11, **caractérisé par le fait que** la formation du groupe d'ester se fait en contactant un agent alkylant avec un pétrole brut résiduel obtenu à partir de l'acidification de l'extrait concentré, extraction de l'extrait acidifié avec un solvant organique, séparation de la phase de solvant organique et enlèvement du solvant.

13. Processus selon la revendication 12, **caractérisé par le fait que** le solvant organique est de l'acétate d'éthyle.

14. Processus selon une des revendications 7 à 13, **caractérisé par le fait que** l'agent alkylant est sélectionné à partir d'un diazoalkyle C1- à C12-.
